# EUROPEAN PATENT APPLICATION

(11) **EP 1 709 964 A2**
(43) Date of publication of application: **11.10.2006**
(21) Application number: 06251929.3
(22) Date of filing: 05.04.2006
(51) Int. Cl.: A61K 31/404, A61K 31/381, A61K 31/343, C07D 307/83, C07D 405/06, C07D 407/06, C07D 409/06, A61P 17/00, A61P 17/02

(54) **A method of cosmetic depigmentation care by applying at least one aurone**

(30) Priority: 06.04.2005 FR 0503409; 12.05.2005 US 127594
(71) Applicant: ENGELHARD LYON, 69007 Lyon (FR)
(72) Inventor: Perrier, Eric, 38138 Les Cotes D'Arey (FR); Okombi, Sabrina, 38100 Grenoble (FR); Rival, Delphine, 69360 Ternay (FR); Boumendjel, Ahcene, 38240 Meylan (FR); Mariotte, Anne-Marie, 38870 Saint Siméon de Bressieux (FR)
(74) Representative: Fisher, Adrian John

(57) **Abstract**

At least one aurone or a natural or synthetic derivative of aurone, or an analogue of aurone, in which the independent phenyl ring can be substituted by a heterocycle of pyrrole, imidazole, triazole, pyridine, furan, or thiophene type, is disclosed as a cosmetic agent, or as an active substance, for the manufacture either of a cosmetic composition, or of a pharmaceutical composition, notably a dermatological composition, having a melanogenesis-inhibiting activity or a depigmenting activity, or an anti-tyrosinase activity.

## Description

### Field of the invention

The present invention essentially relates to the use of at least one aurone, in cosmetics or for the manufacture of a pharmaceutical composition, notably a dermatological composition, having depigmenting activity or having a melanogenesis-inhibiting effect.

The invention also covers cosmetic compositions or pharmaceutical compositions, notably dermatological compositions, which have depigmenting activity or a melanogenesis-inhibiting effect, and which are thus obtained.

The invention also covers a method of cosmetic care or a method of therapeutic treatment of depigmentation which uses the aurones as agents which are active with regard to depigmentation.

In order to fight against the sun's rays, the skin possesses different cells which are particularly adapted to this function : melanocytes. During a complex process, melanogenesis, these cells produce a dark pigment, melanin, the effect of which is to protect the skin structures and to increase the time necessary to contract sunburn. All the melanins are not however protective and in particular, a form of melanin exists, which is called phaeomelanine, which is extremely phototoxic. This molecule, which is capable, like all melanins, of reacting with certain forms of free radicals, causes the formation of free radicals which are even more toxic, and which can cause irreversible damage to the genetic material of the keratinocytes. Furthermore, certain disorders which are linked to a dysfunction of the melanisation unit, can cause a hyper-pigmentation which is sometimes particularly unaesthetic. Thus, the use of inhibitors of the syntheses of melanin is particularly interesting in cosmetics, not only for applications in which a veritable depigmentation is sought after, as in the case of whitening of strongly-pigmented skin, or the inhibition of hyper-pigmentation in certain unaesthetic aspects for example, but also for application which seek to lighten the tint, to give brightness to the skin, luster to the surface tissues. This inhibition of melanin synthesis can also be particularly interesting within the context of therapeutic treatment for treating a veritable pathology.

### STATE OF THE ART

Aurones are natural molecules which belong to the family of flavonoids, and which are structurally isomers of flavones (Boumendjel A., Current Medicinal Chemistry, 2003,10, 2621-2630).

Aurones are broadly widespread in the plant kingdom, particularly in fruits and flowers in which they contribute to their coloration. The Table below contains a non-exhaustive list of natural aurones which are found in plants :

| **Aurone name** | **Family** | **Species** | **Part** |
|---|---|---|---|
| Hispidol | Leguminous | Glycine max, Lygos raetam | seedling |
| Sulfuretin | Composite | Bidens tripartita,Dahlia variabilis, Cosmos sulfureus | Flowers |
| Sulfurein | Asteraceae | Coreopsis bigelovii, Zinnia linearis | Flowers |
| | Anacardiaceae | Cotinus coggygria | |
| Palasitrin | Leguminous | Butea frondosa | |
| Licoagroaurone | Leguminous | Glycyrrhiza glabra | Root |
| Leptosidin | Asteraceae | Corepsis grandiflora | |
| Leptosin | Asteraceae | Coreopsis lanceolata, Coreopsis tinctoria | Flowers |
| | Ericaceae | Vaccinium oxycoccus | Fruit |
| | Leguminous | Flemengia strobilifera | Leaf |
| 4,4',6-trihydroxyaurone | Liliaceae | Asparagus gonocladus | |
| | Rutaceae | Limonium sp | |
| 4,4'-Dihydroxy-6-O-rhamnosylaurone | Leguminous | Pterocarpus marsupium | Flowers |
| Caulesauroneside | Aristolochiaceae | Asarum longerhizomatosum | |
| Antiarone A | Clusiaceae | Antiaris toxicara | Root |
| Antiarone B | Clusiaceae | Antiaris toxicara | Root |
| Aureusidin | Scrophulariaceae | Antirrhinum majus | Flowers |
| | Rutaceae | Citrus medica | |
| | Cyperaceae | Cyperus, Eleacharis, Ptilanthelium, Schoenus, Gahnia | |
| Aureusin | Scrophulariaceae | Antirrhinum majus, Linaria maroccana | Flowers |
| | Rubiaceae | Mussaenda hirsutissima | Flowers |
| Cernuoside | Rubiaceae | Mussaenda hirsutissima | Flowers |
| | Oxalidaceae | Oxalis cernua | |
| | Rutaceae | Limonium bonduellii | |
| Aureusidin 4,6-diglucoside | Rubiaceae | Mussaenda hirsutissima | Flowers |
| Bracteatin | Asteraceae | Helichrysum bracteatum | Flowers |
| | Scrophulariaceae | Antirrhinum nuttalianum, Linaria maroccana | Flowers |
| Bractein | Asteraceae | Helichrysum bracteatum | Flowers |
| Bracteatin-6-glucoside | Scrophulariaceae | Antirrhinum majus, Linaria sp | Flowers |
| Maritimetin | Composite | Coreopsis maritima, C gigantea, C tinctoria | Flowers |
| | | Baeria chrysostoma, Bidens bipinnata | Flowers |
| Maritimein | Composite | Coreopsis maritima, C tinctoria, Baeria chrysostoma | Flowers |
| | Asteraceae | Zinia linearis | Flowers |
| Hamiltrone | Annonaceae | Ulvaria hamiltonii | Leaves |
| 4,6,4'-trihydroxy-7-methyl-4-O-rhamnosylaurone | Leguminous | Pterocarpus marsupium | Flowers |

While flavones, chalcones, flavonols and isoflavones are widely studied for their therapeutic functions, the biological activity of aurones is only beginning to be investigated. Since aurones are in fact less abundant in nature than flavones, these natural molecules have hitherto given rise to less interest. Moreover, due to their role of pigmentation of plants, aurones have been described as phytoalexins, used by the plant as a defense agent against various infections.
In terms of biological activity, aurones are described in the field of cancer for their various modes of action :
- aurones have the capacity to bind to glycoprotein-P, which are transmembrane transporters which enable the mediation of anti-cancer drugs
- aurones inhibit Cyclines kinase,
- aurones interact with adenosine receptors, and
- aurones inhibit telomerase.

Finally, aurones are used for their anti-oxidant properties (particularly glycosylated aurones) by minimizing radical damage which implicates DNA.

Aurones are also known for treating parasite and microbial infections. They have an anti-hormone activity and have been used in certain patients suffering from diabetes for their capacity to inhibit the Maillard reaction.

Aurones, like other flavonoids, originate from pivot precursors which are chalcones. In this case, the production of aurones from chalcones involves an oxidative cyclization, on the condition that these latter molecules have a hydroxyl in position 2' (Nakayama, J. of Bioscience and Bioengineering, 2002, 94 (6), 487-491).

### AIMS OF THE INVENTION

The main aim of the invention is to provide a novel formulation of a cosmetic composition and/or pharmaceutical composition, notably a dermatological composition, which has a good depigmenting activity and/or a good melanogenesis-inhibiting activity.

Another main aim of the invention is to provide a novel formulation of a cosmetic composition and/or pharmaceutical composition, notably a dermatological composition, which has a good anti-tyrosinase activity.

Another main aim of the invention is to provide a method of cosmetic depigmentation care or a method of therapeutic treatment of depigmentation.

Finally, a main aim of the invention is to attain these goals in a simple, safe and reliable manner which can be used on an industrial and cosmetic, pharmaceutical, notably dermatological, scale.

### SUMMARY OF THE INVENTION

The invention enables the aims set forth above to be attained by the demonstration of a novel biological activity of aurones and derivatives, since it is demonstrated by the present invention that aurones have a good depigmenting activity and/or a good melanogenesis-inhibiting activity, particularly, they are strong inhibitors of human tyrosinase, which enzyme is involved in the synthesis of melanogenesis.

This activity, which is particularly unexpected, is not described in the literature and patents in relation to the use of aurones.

### DETAILED DESCRIPTION OF THE INVENTION

The present invention relates, according to a first aspect, to the use of at least one aurone, aurone derivative, or aurone analogue, comprising an independent phenyl ring in which the independent phenyl ring can be replaced by a heterocycle of pyrrole, imidazole, triazole, pyridine, furan, or thiophene type, as cosmetic agent, or as active substance, for the manufacture either of a cosmetic composition, or of a pharmaceutical composition, notably a dermatological composition, having a melanogenesis-inhibiting activity or a depigmenting activity, or an anti-tyrosinase activity.

According to a particular embodiment of the invention, this use is characterised in that the aurone has the general chemical formula described below : 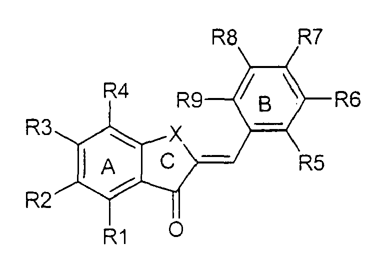 wherein X can represent an oxygen atom (O), a nitrogen atom (N) or a sulfur atom (S),
wherein R1, R2, R3, R4, R5, R6, R7, R8, R9 can represent, independently of each other : H ; a halogen ; a hydroxyl (OH) ; an ester ; a salified or non-salified C1-12 acid ; an amide ; a sulfonamide ; a nitro ; a basic or salified I, II or III amine ; a cyano ; a thiol ; a sugar (O-heteroside or C-heteroside) ; a linear or branched C1-12 alkyl chain ; a linear or branched C1-12 alkoxy chain ; a linear or branched C1-12 alkenyl chain (e.g. prenyl) ; C1-12 alkenyloxy ; a thioalkyl chain ; an alkyl or aromatic type ring or heterocycle ; a salified or non-salified sulfate ; a salified or non-salified sulfonate ; a salified or non-salified phosphate ; an esterified or non-esterified phosphate ; an esterified or non-esterified phosphonate ; a salified or non-salified phosphonate, a silanol group of formula : 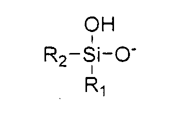 in which R1, R2 can represent, independently of each other : hydroxyl, methyl, ethyl, phenyl, *p*-methylphenyl, *p*-methoxyphenyl, or tert-butyl.

According to another embodiment, the present invention also relates to the use of a derivative of aurone of auronol, azaaurone or thioaurone type, of general formula : 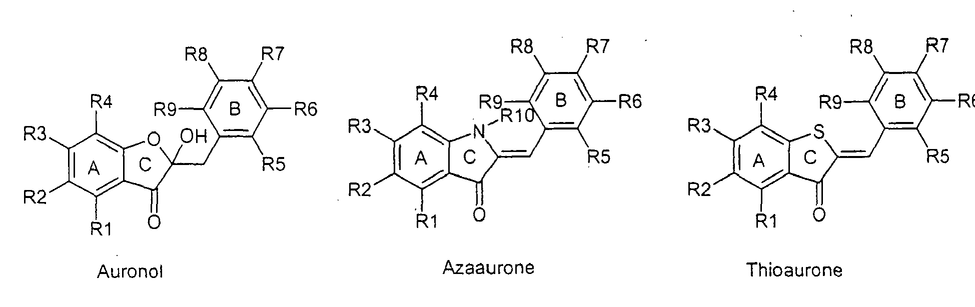 wherein R1 to R9 can represent, independently of each other, the groups cited above. R10 can be a linear or branched alkyl chain or acyl group having 1 to 12 carbon atoms.

According to another embodiment, this invention also relates to the use of dimers of aurones. Example : disulfuretin, biaureusidin, the general formula of which is described below.

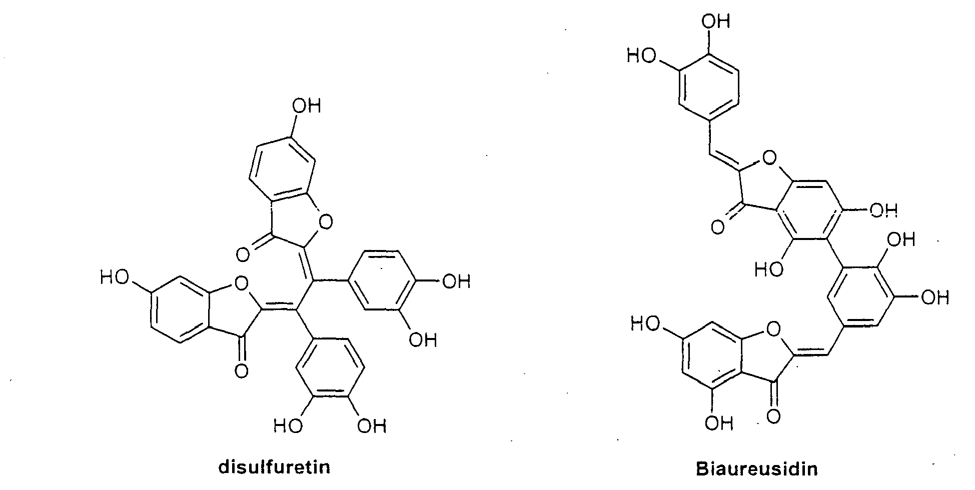

According to yet another embodiment, this invention relates also to the use of the analogues of aurones in which the phenyl ring B can be replaced by a heterocycle of pyrrole, imidazole, triazole, pyridine, furan, or thiophene type.

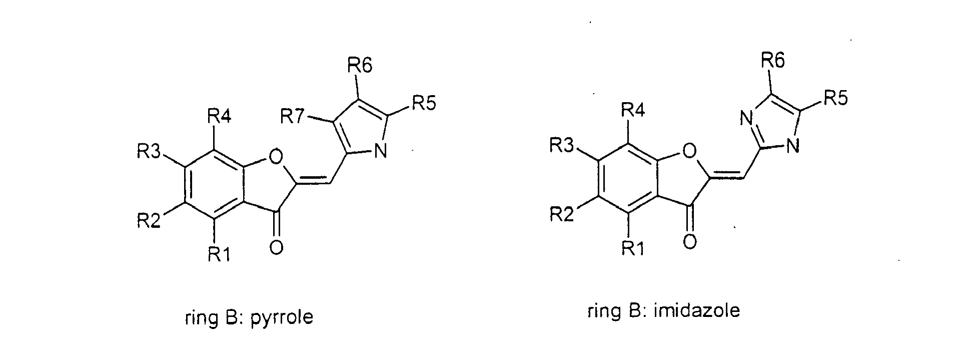

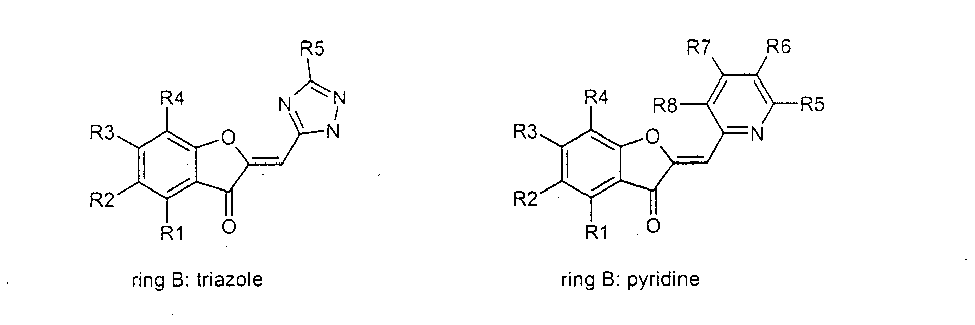

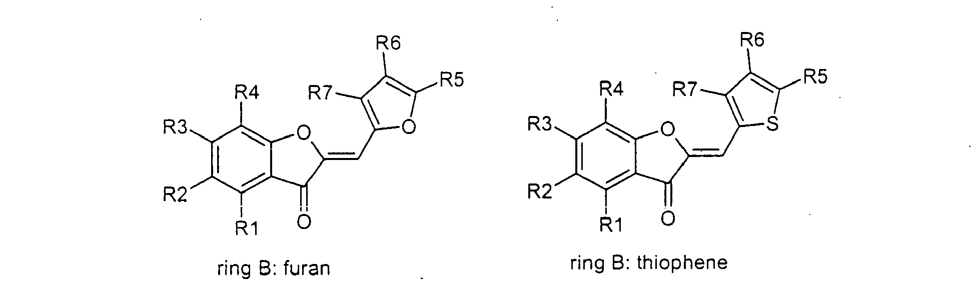

According to various examples of embodiments, the invention also relates to the use of an aurone, of its derivative or of its analogue selected from 2', 4', 6'-Trihydroxy-2-chloroacetophenone (R1 = OH,), 2',4'-Dihydroxy-2-chloroacetophenone (R1 = H) ; 4,6-Dihydroxybenzofuran-3(2H)-one (R1 = OH) ; 6-Hydroxybenzofuran-3(2H)-one (R1 = H); (Z)-2-Benzylidene-6-Hydroxybenzofuran-3(2H)-one; (*Z*)-2-(4'-Ethyl)Benzylidene-6-Hydroxybenzofuran-3(2H)-one; (*Z*)-2-(2'-Ethyl)Benzylidene-6-Hydroxybenzofuran-3(2H)-one; (*Z*)-2-(4'-hydroxy)Benzylidene-6-Hydroxybenzofuran-3(2H)-one; 4,6-Dimethoxybenzofuran-3(2H)-one ; (*Z*)-2-Benzylidene-4,6-dimethoxybenzofuran-3(2H)-one ; (*Z*)-4,6-dimethoxy-2-(4'-hydroxybenzylidene)benzofuran-3(2H)-one ; (*Z*)-4,6-dimethoxy-2-(4'-ethylbenzylidene)benzofuran-3(2H)-one; (*Z*)-4,6-dimethoxy-2-(4'-methylthiobenzylidene)benzofuran-3(2H)-one ; (*Z*)-2-(4-cyanobenzylidene)-4,6-dimethoxybenzofuran-3(2H)-one ; (*Z*)-4,6-dimethoxy-2-(4'-sulfonatebenzylidene)benzofuran-3(2H)-one ; (*Z*)-4,6-dimethoxy-2-(2',4'-disulfonatebenzylidene)benzofuran-3(2H)-one ; (*Z*)-4,6-dimethoxy-2-(4'-methylbenzylidene)benzofuran-3(2H)-one ; (*Z*)-4,6-dimethoxy-2-(4'-propylbenzylidene)benzofuran-3(2H)-one ; (*Z*)-4,6-dimethoxy-2-(4'-isopropylbenzylidene)benzofuran-3(2H)-one ; (*Z*)-2-(4'-butylbenzylidene)-4,6-dimethoxybenzofuran-3(2H)-one; (*Z*)-2-Benzylidene-(4,6-dihydroxy) benzofuran-3(2H)-one ; (*Z*)-4,6-Dihydroxy-2-(4'-ethoxybenzylidene)benzofuran-3(2H)-one ; (*Z*)-4,6-Dihydroxy-2-(4'-hydroxybenzylidene)benzofuran-3(2H)-one ; (2)-4,6-Dihydroxy-2-(3-methoxy-4-hydroxybenzylidene)benzofuran-3(2H)-one ; 4,6-Di-*O*-MEMbenzofuran-3(2H)-one ; 2,6-Diacetoxy-α-bromoacetophenone ; 4-Hydroxybenzofuran-3(2H)-one ; or (Z)-2-(4'-Hydroxybenzylidene)-4-Hydroxybenzofuran-3(2H)-one.

According to still other advantageous examples of embodiments of the invention, the aurone, its derivative or its analogue is selected from (*Z*)-4,6-Dihydroxy-2-(4-methoxybenzylidene)benzofuran-3(2H)-one ; (*Z*)-4,6-Dihydroxy-2-(4'-ethoxybenzylidene)benzofuran-3(2H)-one ; (*Z*)-4,6-Dihydroxy-2-(4'-hydroxybenzylidene)benzofuran-3(2H)-one; and (Z)-4,6-Dihydroxy-2-(3-methoxy-4-hydroxy)benzylidene)benzofuran-3(2H)-one.

According to yet another particular embodiment of the invention, a topical use is made with the aurones, their derivatives and analogues according to the invention, on the skin, the hair or the integuments, notably for an activity of inhibiting human tyrosinase.

According to yet another particular embodiment of the invention, an activity is realized, with the aurones, their derivatives and analogues according to the invention, of inhibiting human tyrosinase originating from normal human melanocytes from donors of light, brown and black phototype.

According to an advantageous embodiment variant, the aurone, its derivative or its analogues, is of natural origin, or is obtained by chemical synthesis, or is present in a plant extract which naturally contains this aurone, its derivative or analogue.

In accordance with a second aspect, the invention also covers cosmetic compositions or pharmaceutical compositions, notably dermatological compositions, having depigmenting activity or having a melanogenesis-inhibiting effect, characterised in that they comprise, as one of these agents having depigmenting activity or melanogenesis-inhibiting activity, at least one aurone, or one of its derivatives or analogues as defined above, optionally mixed with a cosmetically, pharmaceutically acceptable, notably dermatologically acceptable, excipient, vehicle or support.

In accordance with a third aspect, the invention also covers, as a novel product, an aurone, or a derivative of aurone, of the following chemical formula : 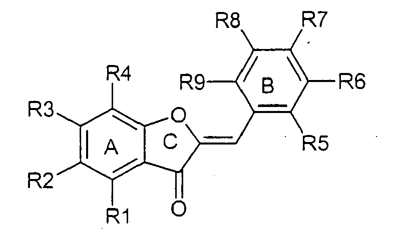 in which the R1 to R9 groups are as defined above, provided that at least one group from R5 to R9 is selected from a linear or branched C1-12 alkyl chain ; a linear or branched C1-12 alkoxy chain ; a C1-C12 thioalkyl group, a sulfonyl group, and a cyano group ;
or an analogue of aurone, of the following chemical formula : 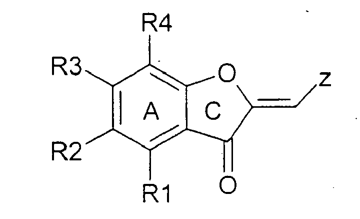 in which the R1, R2, R3, R4 groups are as defined above, and Z represents a group comprising a heterocycle of pyrrole, pyridine, furan, or thiophene type, which is non-substituted or substituted with a R5, R6, R7 group and optionally an R8 group, as defined above, preferably at least one group from R5 to R8 is selected from a linear or branched C1-12 alkyl chain ; a linear or branched C1-12 alkoxy chain , a C1-C12 thioalkyl group, a sulfonyl group, and a cyano group.

In accordance with a fourth aspect, the invention further covers a method of cosmetic depigmentation care, characterised in that it comprises topically applying, onto at least one area of the skin, the hair or the integuments, which is concerned by the depigmentation, an amount which is effective for carrying out said depigmentation of at least one aurone, or one of its derivatives or analogues as defined above, optionally mixed with a cosmetically acceptable excipient, vehicle or support.

In accordance with a fifth aspect, the invention further covers a method of therapeutic treatment of depigmentation, characterised in that it comprises topically applying, onto at least one area of the skin, the hair or the integuments, which is concerned by a pathology of pigmentation, an amount which is effective for carrying out a depigmentation of said zone, of at least one aurone, or one of its derivatives or analogues as defined above, optionally mixed with an excipient, vehicle or support, which is pharmaceutically, notably dermatologically, acceptable.

The term «pathology of pigmentation» is understood as meaning the pigmentation of said area of the skin, the hair or the integuments, is such that it constitutes a pathology to be treated medically. It may be a hyper-pigmentation, a keratosis, etc. as is well-known to the person skilled in the art.

Within the context of the invention, it has been demonstrated in an entirely unexpected manner that these aurones, their derivatives and analogues, were capable of inhibiting tyrosinase from mushroom and tyrosinase from human melanocytes ; and of thus constituting precious cosmetic or pharmaceutical agents, notably precious dermatological agents, for carrying out a good depigmenting activity and/or a good melanogenesis-inhibiting activity and/or a good anti-tyrosinase activity.

Other aims, features and advantages of the invention will appear clearly in the light of the explanatory description which follows made with reference to several examples of realization of synthesis of aurones, of their derivatives and of their analogues, as well as their biological evaluation, as well as with reference to the annexed Figures which represent the results in the form of graphs in columns.

### DESCRIPTION OF THE FIGURES

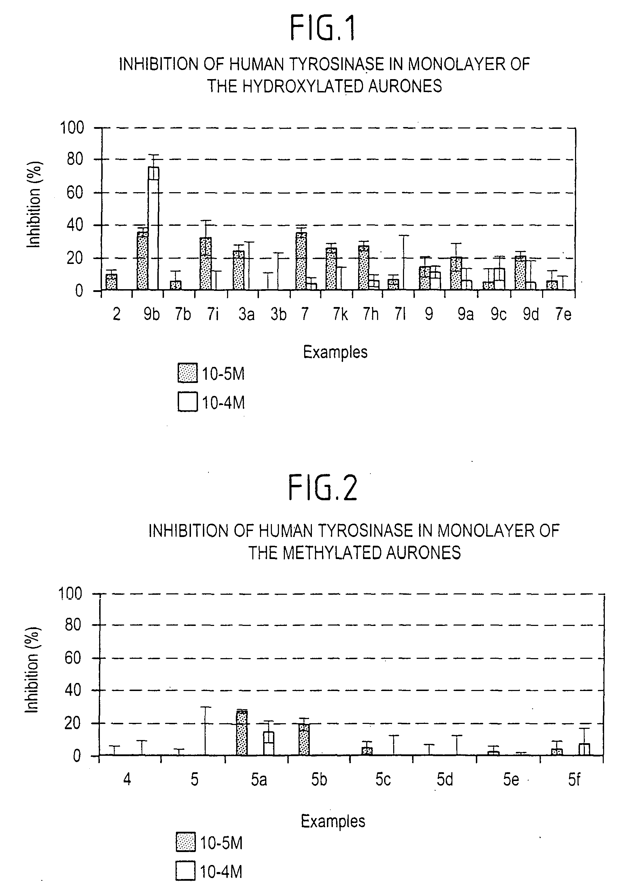

Figure 1 represents a graph of the percentage inhibition of the activity of human tyrosinase, up the ordinate, with, along the abscissa, the examples tested at two different concentrations, 10-5 M and 10-4 M, respectively, the subject of the tests of Example 15, which is carried out with aurones hydroxylated on ring A, shown in Table 3 ;
Figure 2 represents the graph of the percentage inhibition of the activity of human tyrosinase, up the ordinate, with, along the abscissa, the examples tested at two different concentrations, 10-5 M and 10-4 M respectively, subject of the tests of Example 15, with aurones methylated on ring A, shown in Table 4 ;

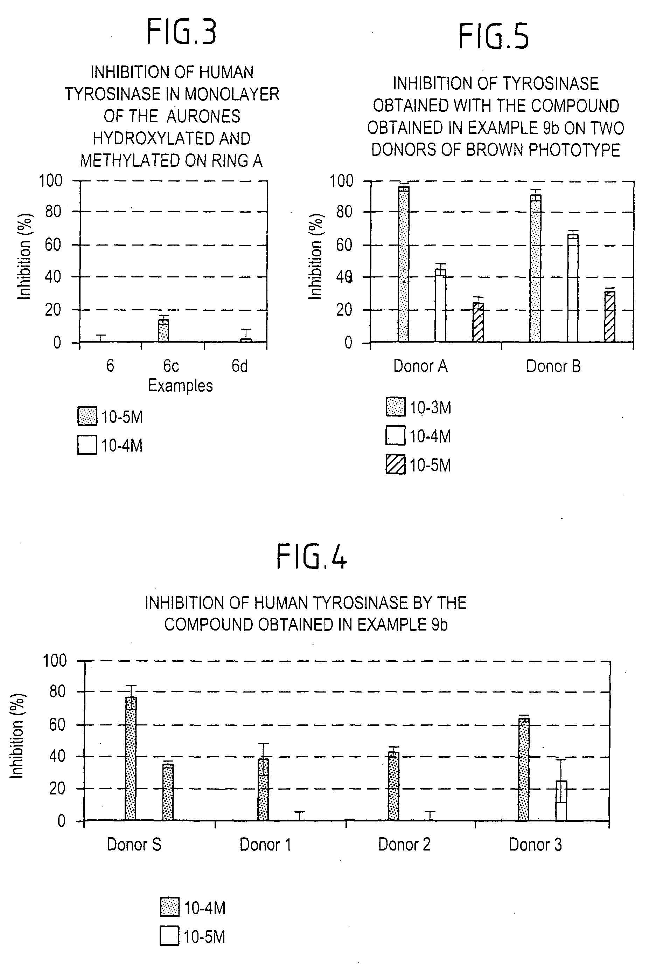

Figure 3 represents the graph of the percentage inhibition of the activity of human tyrosinase, up the ordinate, with, along the abscissa, the examples tested at two different concentrations, 10-5 M and 10-4 M respectively, subject of the tests of Example 15, with aurones hydroxylated and methylated on ring A, shown in Table 5 ;
Figure 4 represents the graph of the percentage inhibition of the activity of human tyrosinase, up the ordinate, with, along the abscissa, the results obtained for four different donors for the compound obtained in Example 9b tested at two different concentrations, 10-5 M and 10-4 M respectively, subject of the tests of Example 16, these results are also shown in Table 6.
Figure 5 represents the graph of the percentage inhibition of the activity of human tyrosinase, up the ordinate, with, along the abscissa, the results obtained for two donors of brown and black phototype for the compound obtained in Example 9b tested at three different concentrations, 10-5 M, 10-4 M and 10-3M, respectively, subject of the tests of Example 17, these results are also shown in Tables 7 and 8.

In the Examples of syntheses which follow, all percentages are given by weight, the pressure is atmospheric pressure, the temperature is in degrees Celsius, unless indicated otherwise.

### SYNTHESIS OF THE AURONES AND BIOLOGICAL EVALUATION

### Example 1 of the invention : Synthesis of 2', 4', 6'-Trihydroxy-2-chloroacetophenone (R1 = OH) and 2',4'-Dihydroxy-2-chloroacetophenone (R1 = H) :

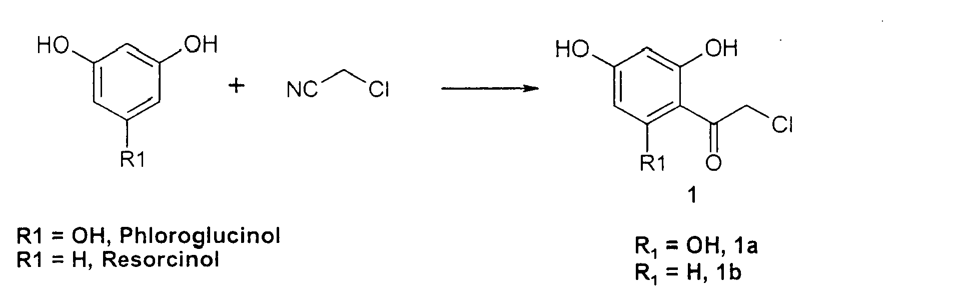

To a solution of phloroglucinol (5 g, i.e. 39.6 mmol) or resorcinol (5 g, *i.e.* 45.4 mmol) in ether (150 mL), are added, successively, chloroacetonitrile (1eq) and zinc chloride (0.1eq). The whole is cooled to 0°C and is allowed to react with gaseous hydrochloric acid for one hour. The precipitate formed is recovered by filtration and is washed with ether.

The precipitate is dissolved in 1N hydrochloric acid and the solution obtained is held under reflux for one hour. The medium is brought to ambient temperature and the orange precipitate formed is recovered and washed with water.

### Example 2 of the invention : Synthesis of 4,6-Dihydroxybenzofuran-3(2H)-one (R1 = OH) and 6-Hydroxybenzofuran-3(2H)-one (R1 = H) :

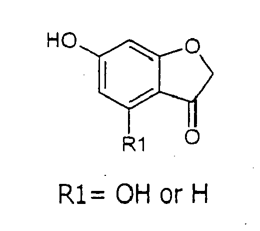

The compounds obtained in Example 1, (R1=OH or H) are placed in suspension in methanol (100 mL). Sodium methoxide (2.5eq) is then added. The solution obtained is agitated for about 10 minutes in the cold and is then held under reflux for 1h 30. After return to ambient temperature, the medium is acidified with a solution of HCl and is evaporated. The residue obtained is taken up into ethyl acetate. The organic phase is washed with water, a saturated sodium chloride solution, is dried over sodium sulfate and is evaporated to dryness, to give the expected product as an orange powder.

### Example 3 of the invention : Synthesis of (Z)-2-Benzylidene-6-Hydroxybenzofuran-3(2H)-one :

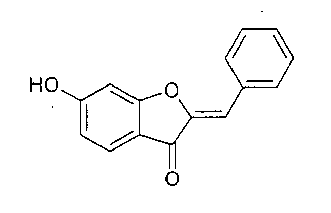

To the compound obtained in Example 2 and wherein R1= H (1.0 mmol), in methanol, are added, successively, a 50% solution of potassium hydroxide (1.5 mL) and benzaldehyde (1.5 eq). The solution obtained is held under reflux for 1 hour. After return to ambient temperature, the methanol is evaporated off and the residue obtained is taken up into water. The solution obtained is acidified. A precipitate appears which is recovered by filtration and washed with water. The product is obtained as an amorphous powder after silica gel column chromatography.

The method described above can be applied by replacing the benzaldehyde :
by p-Ethylbenzaldehyde ; in this case, the molecule of Example 3 is obtained with an ethyl group in 4' and becomes Example 3a,
by 2-ethylbenzaldehyde ; in this case, the molecule of Example 3 is obtained with an ethyl group in 2' and becomes Example 3b,
by 4-hydroxylbenzaldehyde ; in this case the molecule of Example 3 is obtained with a hydroxyl group in 4' and becomes Example 3c.

### Example 4 of the invention : Synthesis of 4,6-Dimethoxybenzofuran-3(2H)-one :

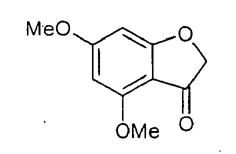

To the compound obtained in Example 2 and wherein R1= OH, dissolved in DMF, is added potassium carbonate (3 eq) and methyl iodide (3 eq). The mixture obtained is heated for 1 hour (150°C). After return to ambient temperature, the medium is mixed with water and is extracted with ethyl acetate. The organic phase is dried and is evaporated to dryness. The product is isolated as a yellow powder after purification by silica gel column chromatography.

### Example 5 of the invention : Synthesis of (Z)-2-Benzylidene-4,6-dimethoxybenzofuran-3(2H)-one :

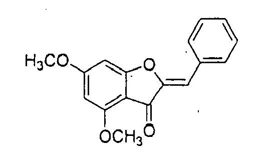

To a solution of the compound obtained in Example 4 of the invention (0.77 mmol), dissolved in methanol, are added, successively, a 50% solution of potassium hydroxide (1.5 mL) and benzaldehyde (1.5 eq). The solution obtained is held under reflux for 1 hour. After return to ambient temperature, the methanol is evaporated off and the residue obtained is taken up with water. The solution obtained is acidified and then extracted 3 times with ethyl acetate. The organic phase is dried over sodium sulfate and is evaporated under vacuum.

The product is obtained as a pale yellow powder after silica gel column chromatography.

The method described above can be applied by replacing the benzaldehyde :
by *p*-hydroxybenzaldehyde ; in this case the molecule of Example 5 is obtained with a hydroxyl group in 4' (Example 5a),
by *p*-ethylbenzaldehyde ; in this case the molecule of Example 5 is obtained with an ethyl group in 4' (Example 5b),
by *p*-methylthiobenzaldehyde ; in this case the molecule of Example 5 is obtained with a thiomethyl group in 4' (Example 5c),
by *p*-cyanobenzaldehyde ; in this case the molecule of Example 5 is obtained with a cyano group in 4' (Example 5d),
by *p*-sulfonatebenzaldehyde ; in this case the molecule of Example 5 is obtained with a sulfonate group in 4' (Example 5e),
by 2,4-disulfonatebenzaldehyde ; in this case the molecule of Example 5 is obtained with two sulfonate groups in 2' and 4' (Example 5f),
by *p*-tolualdehyde ; in this case the molecule of Example 5 is obtained with a methyl group in 4' (Example 5g),
by *p*-propylbenzaldehyde ; in this case the molecule of Example 5 is obtained with a propyl group in 4' (Example 5h),
by *p*-isopropylbenzaldehyde ; in this case the molecule of Example 5 is obtained with an isopropyl group in 4' (Example 5i),
by *p*-butylbenzaldehyde ; in this case the molecule of Example 5 is obtained with a butyl group in 4' (Example 5j),
by *p*-tert-butylbenzaldehyde ; in this case the molecule of Example 5 is obtained with a tert-butyl group in 4' (Example 5k),
by 2'-ethylbenzaldehyde ; in this case the molecule of Example 5 is obtained with an ethyl group in 2' (Example 5l),
by 2-furaldehyde ; in this case the molecule of Example 5 is obtained with a furan ring replacing ring B (Example 5m),
by 5-Ethyl-2-furaldehyde ; in this case the molecule of Example 5 is obtained with a 5-Ethylfuran ring replacing ring B (Example 5n),
by 4,5-Dimethyl-2-furaldehyde ; in this case the molecule of Example 5 is obtained with a 4,5-Dimethylfuran ring replacing ring B (Example 5o),
by 2-thiophenecarboxaldehyde ; in this case the molecule of Example 5 is obtained with a thiophene ring replacing ring B (Example 5p),
by 5-Methyl-2-thiophenecarboxaldehyde ; in this case the molecule of Example 5 is obtained with a 5-Methylthiophene ring replacing ring B (Example 5q),
by 5-Ethyl-2-thiophenecarboxaldehyde ; in this case the molecule of Example 5 is obtained with a 5-Ethylthiophene ring replacing ring B (Example 5r),
by Pyrrole -2-carboxaldehyde ; in this case the molecule of Example 5 is obtained with a pyrrole ring replacing ring B (Example 5s),
by 3,5-Dimethylpyrrole-2-carboxaldehyde; in this case the molecule of Example 5 is obtained with a 3,5-Dimethylpyrrole ring replacing ring B (Example 5t).

### Example 6 of the invention : Synthesis of (Z)-2-Benzylidene-4-hydroxy-6-methoxybenzofuran-3(2H)-one :

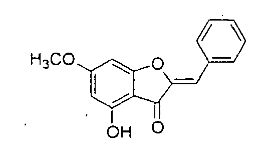

To the compound obtained in Example 5 in dichloromethane is added a molar solution of boron tribromide in dichloromethane (4 eq). The mixture obtained is agitated for 20 hours at ambient temperature. The mixture is then poured into iced water and the whole is extracted with ethyl acetate. The organic phase is dried and evaporated to dryness. The product is obtained as a yellow powder after purification by silica gel column chromatography.

The method described above can be applied to the molecules obtained in Examples 5c (which becomes 6c) and 5d (which becomes 6d).

### Example 7 of the invention : Synthesis of (Z)-2-Benzylidene-(4,6-dihydroxy) benzofuran-3(2H)-one :

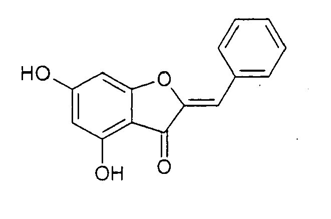

To the compound obtained in Example 5 of the invention, dissolved in dichloromethane, is added boron tribromide (BBr₃; in excess) ; the mixture is then agitated at ambient temperature for 20 hours. Iced water is then poured dropwise into the medium and the suspension thus formed is extracted with ethyl acetate. The organic phase is dried and evaporated to dryness to give the expected product as an amorphous precipitate.

The method described above can be applied to the molecules obtained in Examples 5b , 5e, 5g, 5h, 5i, 5j, 5k, 5l, 5m, 5n, 5o, 5p, 5q, 5r, 5s, and 5t, which become Examples 7b, 7e, 7g, 7h, 7i, 7j, 7k, 7l, 7m, 7n, 7o, 7p, 7q, 7r, 7s, and 7t, respectively.

### Example 8 of the invention : Synthesis of 4,6-Di-O-MEMbenzofuran-3(2H)-one :

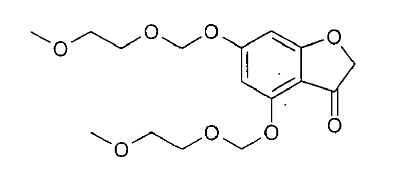

The compound of Example 2, wherein R1=OH (36 mmol) to which is added *N,N*-diisopropylthylamine (2 eq) in anhydrous DMF, is cooled with the aid of an ice bath. 2-methoxyethoxymethyl chloride, (2 eq) is added dropwise and the solution obtained is agitated for 30 minutes at ambient temperature. The medium is then poured into 100 mL of water and is extracted with ethyl acetate. The organic phase is dried and evaporated to dryness. The product is obtained as a white oil after purification by silica gel column chromatography.

### Example 9 of the invention : Synthesis of (Z)-4,6-Dihydroxy-2-(4-methoxybenzylidene)benzofuran-3(2H)-one :

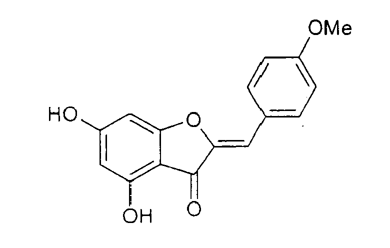

To a solution of the compound obtained in Example 8 (1.02 mmol) in methanol are added successively, a 50% solution of potassium hydroxide (3.5 mL) and *p*-anisaldehyde (1.5 eq). The solution obtained is held under reflux for 1 hour. After return to ambient temperature, the methanol is evaporated off and the residue obtained is taken up into water. The solution obtained is extracted with ethyl acetate. The organic phase is dried and evaporated to dryness. The product obtained is taken up into methanol. To the solution obtained is added a 1M solution of hydrochloric acid in ether. The mixture is agitated at about 60°C for 2 hours. After return to ambient temperature, the medium is mixed with water and extracted with dichloromethane. The organic phase is washed successively with water, a saturated sodium chloride solution, dried and evaporated to dryness. The product is obtained as a yellow powder after purification by silica gel column chromatography.

The method described above can be applied by replacing the p-anisaldehyde by :
*p*-ethoxybenzaldehyde ; in this case an ethoxy group is obtained in 4' is obtained (Example 9a),
*p*-hydroxybenzaldehyde, in this case a hydroxyl group is obtained in 4' (9b) or in 2'(9c),
vanillin, in this case a methoxy group is obtained in 3' and a hydroxyl group in 4' (9d).

### Example 10 of the invention: Synthesis of 2,6-Diacetoxy-a-bromoacetophenone:

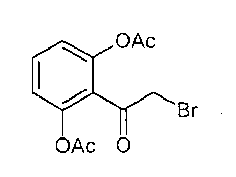

2,6-Dihydroxyacetophenone (3.08 mmol) is dispersed in acetic anhydride and the whole is held under reflux for 4 hours. The medium is poured into iced water and is extracted with ethyl acetate. The organic phase is dried and evaporated to dryness to give an oil which is taken up into anhydrous THF. To the solution obtained is slowly added trimethylphenylammonium bromide (1.2 eq) ; the mixture obtained is agitated for 6 hours at ambient temperature. Water is then added into the medium and the solution obtained is agitated for a few minutes at ambient temperature and then extracted with ethyl acetate. The organic phase is dried and evaporated to dryness. The crude product is obtained as an orange oil and is used in the next steps without purification.

### Example 11 of the invention : Synthesis of 4-Hydroxybenzofuran-3(2H)-one :

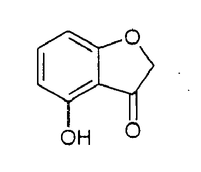

To the compound of Example 10 (380 mg) in methanol is added a 50% aqueous solution of potassium hydroxide (0.5 mL). The whole is held under reflux for about 3 hours. After return to ambient temperature, the methanol is evaporated off under vacuum and the residue obtained is taken up into water and is acidified with the aid of a solution of HCl. The suspension obtained is then extracted with dichloromethane. The organic phase is then dried and evaporated to dryness.

The product is obtained as an off-white precipitate after purification by silica gel column chromatography.

### Example 12 of the invention: Synthesis of (Z)-2-(4'-Hydroxybenzylidene)-4-Hydroxybenzofuran-3(2H)-one :

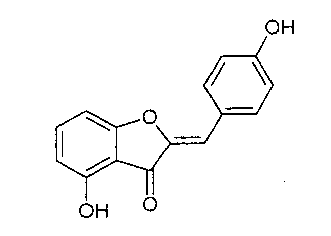

To the compound obtained in Example 11 (0.66 mmol), in methanol, are successively added a 50% potassium hydroxide solution and 4-hydroxybenzaldehyde (1.5 eq). The solution obtained is held under reflux for 3 hours. After return to ambient temperature, the methanol is evaporated off and the residue obtained is taken up into water. The solution obtained is acidified : a yellow precipitate appears which is recovered by filtration, washed with a minimum of water and dried under vacuum. The product is obtained as an orange precipitate after purification by preparative chromatography.

### Biological evaluation of the AURONES

### Example 13 of the invention : In vitro test of the inhibition of isolated tyrosinase by the aurones :

Tyrosinase catalyses the formation of L-dopaquinone, and then dopachrome, from L-Dopa. Dopachrome is a colored compound which is quantifiable by visible spectrophotometry at 490 nm. The use of an active which is capable of modifying the enzymatic activity manifests itself by a variation in the optical density at 490nm.

The ratio of the rates of formation of the dopachrome enables the activations or the inhibitions obtained with the various molecules tested to de determined precisely.

The sample to be tested is incubated in the presence of tyrosinase from mushroom (Sigma) for 5 minutes under agitation. L-DOPA (Sigma), substrate of the tyrosinase, is incubated for 10 minutes in the dark in the presence or the absence of the molecules to be tested. The calculation of the percentage inhibition is made by comparing the OD of the test to the OD of the negative control without molecule. The positive control used is kojic acid (Sigma) at 0.01% =45% ± 5% inhibition.

Within the context of this *in vitro* test, the aurone derivatives were tested at final concentrations of 10⁻⁴M and 10⁻⁵M. The results are described in Table 1.

**Table 1 : Inhibition of tyrosinase from mushroom by various aurones at 490 nm, expressed as percentage inhibition.**

| **Aurones** | **10**^{**-4**}**M** | **10**^{**-5**}**M** |
|---|---|---|
| **Hydroxylated on ring A** | | |
| Example 9b | 23.85 ± 2.42 | 0 ± 1.73 |
| Example 7b | 54.57 ± 3.01 | 14.63 ±6.73 |

| **Methylated on ring A** | | |
|---|---|---|
| Example 5a | 0 ± 1.33 | 0 ± 3.90 |
| Example 5b | 0 ± 4.21 | 0 ± 2.5 |
| Example 5c | 0 ± 1.64 | 1.07 ± 4.37 |
| Example 5 | 0 ± 1.58 | 0 ± 0.28 |
| Example 5d | 0 ± 2.42 | 0 ± 5.47 |

| **Hydroxylated and methylated on ring A** | | |
|---|---|---|
| Example 6c | 23.65 ± 9.36 | 0 ± 5.84 |
| Example 6 | 31.22 ± 3.94 | 0 ± 2.27 |
| Example 6d | 0 ± 3.51 | 0 ± 3.94 |

It results clearly from Table 1, above, that the aurones inhibit the tyrosinase even at low concentrations. The activity measured depends upon the structure of the aurones since the activity is greater when the ring A is preferably hydroxylated.

### Example 14 of the invention : In vitro test of the inhibition of human tyrosinase by the aurones :

Human tyrosinase obtained from melanocyte extracts originating from healthy donors catalyses the formation of L-Dopaquinone from L-Dopa. L-dopaquinone can be quantified by visible spectrophotometry at 490 nm by virtue of a chromogen : 3-methyl-2-benzothiazolinone hydrazone (MBTH).

This reagent traps the o-quinones synthesized by the tyrosinase to give a MBTH-o-quinone compound which is stable and soluble and which possesses a high molar optical density.

Thus, the use of an active which is capable of modifying the enzymatic activity manifests itself by a variation of the OD at 490 nm compared with that obtained in the negative control (100% of activity).

The melanocyte extract is obtained after lysis of the cell membranes of the normal human melanocytes which lysis is carried out by a heat shock. The supernatant is recovered and then incubated with MBTH (Sigma) and L-Dopa (Sigma). The OD at 490 nm measured after 30 minutes is compared for each active tested to that obtained for the control and the percentage inhibition is calculated by comparing the OD of the test (molecule tested) to the OD of the negative control (without molecule). The positive control used is kojic acid at 0.1% (60% ± 5% inhibition).

The results obtained are grouped together in Table 2.

**Table 2 : Inhibition of human tyrosinase by an aurone at 490 nm, results expressed as percentage inhibition.**

| Aurones Hydroxylated on ring A | 10⁻⁴M | 10⁻⁵M |
|---|---|---|
| Example 9b | 17.99 ± 1.6 | 5.62 ± 2.73 |

It results from Table 2 above that the inhibitory activity of the aurones is present but is modest on this particular model of isolated human tyrosinase.

### Example 15 of the invention : Test of inhibition of human tyrosinase studied in a monolayer after application of the actives to be tested on normal human melanocytes :

Normal human melanocytes (originating from abdominal plasty) are sown in a 24-well plate at the rate of 80,000 cells per well. They are cultivated up to confluence and the actives are applied for 24 hours in the culture media. After 24 hours, the media are removed and the melanocytes are picked up by mechanical action. An extraction is made by heat shock and the supernatants are then recovered and then incubated with MBTH (Sigma) and L-Dopa (Sigma). The OD at 490 nm is measured after 30 minutes, and the inhibition of the tyrosinase is calculated by comparing the OD at 490 nm to the protein level (measured in each culture well) of the test with respect to the ratio : OD 490 nm/concentration of protein of the negative control (non-treated control). A percentage of the anti-tyrosinase activity is thus calculated with respect to the non-treated control.

The negative control of the experiment is kojic acid applied at 0.1% on the melanocytes (for a measured inhibition of 20% ± 5%), and the derivatives of aurones were studied at concentrations of 10⁻⁴ M and 10⁻⁵ M. The results obtained are described below :

**Table 3 : Inhibition of human tyrosinase after application of the hydroxylated aurones on normal human melanocytes, results expressed as percentage inhibition (nd : non-determined).**

| Aurones hydroxylated on ring A | 10⁻⁵M | 10⁻⁴M | SD at 10⁻⁵M | SD at 10⁻⁴M |
|---|---|---|---|---|
| Example 2 | 9.69 | nd | 1.47 | nd |
| Example 9b | 35.23 | 75.31 | 2.34 | 8.51 |
| Example 7b | 4.52 | nd | 6.97 | nd |
| Example 7i | 31.86 | 0 | 10.43 | 11.43 |
| Example 3a | 23.9 | 0 | 4.02 | 29.28 |
| Example 3b | 0 | 0 | 10.25 | 22.85 |
| Example 7 | 34.85 | 3.37 | 3.04 | 4.8 |
| Example 7K | 25.1 | 0 | 3.98 | 14.08 |
| Example 7h | 26.88 | 5.43 | 2.93 | 3.43 |
| Example 71 | 6.1 | 0 | 2.79 | 33.92 |
| Example 9 | 14.05 | 10.68 | 6.06 | 3.89 |
| Example 9a | 19.94 | 5.25 | 8.58 | 8.21 |
| Example 9c | 4.32 | 12.85 | 8.92 | 7.52 |
| Example 9d | 20.22 | 3.92 | 3.79 | 13.72 |
| Example 7e | 4.83 | 0 | 6.61 | 8.38 |

It results clearly from Table 3 and Figure 1 that the results obtained show a real efficiency of the hydroxylated aurones on the inhibition of human melanogenesis on the model evaluated. The percentages of inhibition which are observed are very clearly greater than those obtained on a model further away from the living being, namely tyrosinase from mushroom and by direct contact with a tyrosinase extracted from normal human melanocytes.

The hydroxylated aurones thus possess an activity which is particularly unexpected to the person skilled in the art and which is very significant. Preferably, the aurone which is hydroxylated on ring A and having a hydroxyl in position 4' (Example 9b) is particularly active.

**Table 4 : Inhibition of human tyrosinase after application of the methylated aurones on normal human melanocytes, results expressed as percentag einhibition.**

| Aurones methylated on ring A | 10⁻⁵M | 10⁻⁴M | SD at 10⁻⁵M | SD at 10⁻⁴M |
|---|---|---|---|---|
| Example 4 | 0 | 0 | 5.66 | 8.76 |
| Example 5 | 0 | 0 | 2.85 | 19.18 |
| Example 5a | 27.38 | 14.44 | 0.28 | 6.64 |
| Example 5b | 19.38 | nd | 2.66 | nd |
| Example 5c | 4.27 | 0 | 5.33 | 12.41 |
| Example 5d | 0 | 0 | 6.53 | 12.77 |
| Example 5e | 1.44 | 0 | 4.03 | 0.58 |
| Example 5f | 2.88 | 9.36 | 6.11 | 10.04 |

It results from Table 4 and from Figure 2 that the methylated aurones inhibit human tyrosinase, when they are applied on normal human melanocytes, to a lesser degree than the hydroxylated aurones.

**Table 5 : Inhibition of human tyrosinase after application of the hydroxylated and methylated aurones on normal human melanocytes, results expressed as percentage inhibition.**

| Aurones hydroxylated and methylated on ring A | 10-5M | 10-4M | SD at 10-5M | SD at 10-4M |
|---|---|---|---|---|
| Example 6 | 0 | nd | 18.39 | nd |
| Example 6c | 14.19 | nd | 1.59 | nd |
| Example 6d | 0 | 2.31 | 11.93 | 5.02 |

It also results clearly from Table 5 and from Figure 3 that in the same manner as the methylated aurones, the hydroxylated and methylated aurones inhibit the tyrosinase less significantly.

**Table 6 : Inhibition of human tyrosinase after application of the aurone analogues on normal human melanocytes, results expressed as percentage inhibition.**

| **Example** | **Name** | 10-4M | 10-5M | SD 10-4M | SD 10-5M |
|---|---|---|---|---|---|
| 5m | (*Z*)-4,6-Dimethoxy-2-[(furan)methylene]benzofuran-3(*2H*)-one | 19.5 | 6.4 | 2.3 | 5.1 |
| 5n | (*Z*)-4,6-Dimethoxy-2-[(5-ethylfuran)methylene]benzofuran-3(2*H*)-one | 7.8 | 0 | 5.2 | 3.3 |
| 5o | (*Z*)-4,6-Dimethoxy-2-[(4,5-dimethylfuran)methylene]benzofuran-3(*2H*)-one | 23.2 | 12.1 | 0.4 | 3.4 |
| 5p | (*Z*)-4,6-Dimethoxy-2-[(thiophene)methylene]benzofuran-3(*2H*)-one | 31.3 | 6.4 | 0.3 | 3.2 |
| 5q | (*Z*)-4,6-Dimethoxy-2-[(5-methylthiophene)methylene]benzofuran-3(*2H*)-one | 4.8 | 0 | 5.3 | 3.3 |
| 5r | (*Z*)-4,6-Dimethoxy-2-[(5-ethylthiophene)methylene]benzofuran-3(*2H*)-one | 11.9 | 2.1 | 3.5 | 2.3 |
| 5s | (*Z*)-4,6-Dimethoxy-2-[(pyrrole)methylene]benzofuran-3(*2H*)-one | 13.7 | 0 | 3.2 | 3.2 |
| 5t | (*Z*)-4,6-Dimethoxy-2-[(3,5-dimethylpyrrole)methylene]benzofuran-3(*2H*)-one | 5.1 | 0 | 2.1 | 3.1 |
| 7m | (*Z*)-4,6-Dihydroxy-2-[(furan)methylene]benzofuran-3(*2H*)-one | 23.6 | 6.4 | 0.5 | 4.1 |
| 7n | (*Z*)-4,6-Dihydroxy-2-[(5-ethylfuran)methylene]benzofuran-3(*2H*)-one | 28.5 | 13.4 | 1.2 | 3.4 |
| 7o | (*Z*)-4,6-Dihydroxy-2-[(4,5-dimethylfuran)methylene]benzofuran-3(*2H*)-one | 13.6 | 4.5 | 3.4 | 8 |
| 7p | (*Z*)-4,6-Dihydroxy-2-[(thiophene)methylene]benzofuran-3(*2H*)-one | 35.2 | 15.3 | 0.13 | 2.1 |
| 7q | (*Z*)-4,6-Dihydroxy-2-[(5-methylthiophene)methylene]benzofuran-3(*2H*)-one | 16.4 | 7 | 1.3 | 4 |
| 7r | (*Z*)-4,6-Dihydroxy-2-[(5-ethylthiophene)methylene]benzofuran-3(*2H*)-one | 13.8 | 0 | 3 | 2.2 |
| 7s | (*Z*)-4,6-Dihydroxy-2-[(pyrrole)methylene]benzofuran-3(*2H*)-one | 22.8 | 9.1 | 2.4 | 3 |
| 7t | (*Z*)-4,6-Dihydroxy-2-[(3,5-dimethylpyrrole)methylene]benzofuran-3(*2H*)-one | 26.4 | 0 | 2.4 | 3.1 |

It also results clearly from Table 6 that the most of the aurone analogues significantly inhibit human tyrosinase and consequently prevent the formation of melanin and skin pigmentation which is the direct consequence.

### Example 16 of the invention : Test of inhibition of human tyrosinase after application of the compound obtained in Example 9b, on normal human melanocytes originating from various donors.

The compound obtained in Example 9b was tested on melanocytes originating from various donors but of light phototypes according to the method described in Example 15. The donors tested are the following :
- donor S (donor tested in Example 15) : 46 years old
- donor 1 : 40 years old
- donor 2 : 47 years old
- donor 3 : 33 years old
The results obtained are described in Table 6 below and in Figure 4.

**Table 6 : Inhibition of tyrosinase by compound 9b on 4 different donors.**

| Aurone obtained in Example 9b | 10⁻⁴ M | 10⁻⁵ M | SD at 10⁻⁴ M | SD at 10⁻⁵ M |
|---|---|---|---|---|
| Donor S | 75.31 | 35.23 | 8.51 | 1.47 |
| Donor 1 | 37.76 | -9.77 | 10.78 | 15.34 |
| Donor 2 | 43.03 | -7.56 | 2.71 | 13.07 |
| Donor 3 | 63.39 | 24.68 | 2.1 | 13.36 |

The results obtained show a high efficiency of compound 9b on 4 different donors.

### Example 17 of the invention : Test of inhibition of human tyrosinase after application of the compound obtained in Example 9b, on normal human melanocytes originating from various donors of brown and black phototypes.

The compound obtained in Example 9b is tested on cultures of melanocytes originating from 2 donors of brown phototypes and from one donor of negroid phototype. The method applied is that described in Example 15.

The results obtained from the 2 donors of brown phototype are described in Table 7 and in Figure 5.

**Table 7 : Inhibitions of tyrosinase which are obtained with the compound obtained in Example 9b on 2 donors of brown phototype.**

| | Donor A | Donor B | SD donor A | SD donor B |
|---|---|---|---|---|
| 10⁻³ M | 97.51 | 91.8 | 0.74 | 1.89 |
| 10⁻⁴ M | 44.96 | 66.96 | 3.76 | 1.51 |
| 10⁻⁵ M | 25.41 | 31.8 | 3.76 | 1.16 |

The results obtained on the brown phototypes show that the anti-tyrosinase activity is dose-dependent and high. The results obtained with the black phototype (31 years old) is described in Table 8.

**Table 8 : Inhibition obtained for the compound obtained in Example 9b on a donor of black phototype.**

| | Black donor | SD |
|---|---|---|
| 10⁻⁴ M | 65.83 | 3.22 |
| 10⁻⁵ M | 39.51 | 4.97 |

The tyrosinase inhibitory activity originating from black phototype is dose dependent and confirms the results obtained firstly from donors of light phototype and secondly from donors of black phototypes.

### Example 18 of the invention : Study of the cytotoxicity of the aurones :

The cytotoxicity of the actives is studied on normal human melanocytes in a 24-well plate by a determination with PNPP (P-nitrophenyl phosphate), a substance which is converted into p-nitrophenol by the intracellular acid phosphatases of viable cells.

The absorbance of p-nitrophenol at 405 nm is directly proportional to the number of viable cells.

The actives are tested at 2 different concentrations (10⁻⁴ M and 10⁻⁵ M) and are added to the culture medium and are incubated at 37°C for 24 hours. The determination with PNPP is carried out on a cell mat and the results are expressed as a percentage viability with respect to the negative control (non-treated wells).
The results obtained are summarized in Table 9 below :

**Tableau 9 : Percentages viability obtained for the various aurone derivatives on normal human melanocytes (the molecules were tested at 2 concentrations 10⁻⁴ and 10⁻⁵ M.**

| Examples | 10⁻⁴ M | 10⁻⁵ M |
|---|---|---|
| 5 | 83.1 | 98.55 |
| 5d | 98.93 | 96.51 |
| 6 | 56.41 | 121.56 |
| 6d | 85.65 | 119.36 |
| 2 | 103.84 | 126.09 |
| 4 | 106.77 | 85.33 |
| 5a | 104.53 | 90.87 |
| 5b | 13.63 | 99.62 |
| 5c | 80.08 | 136.13 |
| 6c | 20.87 | 116.24 |
| 9b | 90.31 | 128.39 |
| 7b | 63.52 | 143.15 |

The molecules tested are non-cytotoxic when tested at a molar concentration of 10⁻⁴ and 10⁻⁵ M since the percentages viability obtained are greater than 75% viability (tolerated threshold). Only 3 molecules possess a threshold of lower than 75% when tested at 10⁻⁴ M, namely the molecules obtained in Examples 6, 5b and 6c. In order to be evaluated, it will therefore be necessary that these molecules be tested in a monolayer at concentration of less than 10⁻⁴ M (*e*.*g*. 10⁻⁵ M).

### Example 19 of the invention :Study of the specificity of the aurones :

A study is made with molecules known in the literature for their depigmenting activity. These molecules are applied to the model described in Example 15 so as to compare their efficiency with respect to the aurones described above.

It was possible to evaluate vitamin C stabilized by a magnesium phosphate group, or VitC PMg, as well as kojic acid, in our model.

The results obtained are described in Table 10.

**Table 10 : Inhibition of tyrosinase obtained for literature controls.**

| Molecule and concentration tested | Inhibition (%) | SD | cytotoxicity |
|---|---|---|---|
| VitC 3% | 97.77 | 1.15 | cell death |
| VitC 0.3% | 49.28 | 4.86 | cell death |
| VitC 0.03% | -5.68 | 7.3 | no |
| VitC PMG 3% | -3.49 | 1.65 | no |
| VitC PMG 0.3% | 1.55 | 3.28 | no |
| VitC PMG 0.03% | 2.93 | 2.79 | no |
| Kojic acid 0.5% | 25.09 | 3.2 | cell death |
| Kojic acid 0.05% | 14.26 | 4.37 | no |
| Kojic acid 0.005% | 6.84 | 4.32 | no |

The molecules tested revealed to be ineffective in the model of Example 15. No molecule has in fact enabled the human tyrosinase to be inhibited at a threshold which is comparable to that of the aurones and, more particularly, to the molecule obtained in Example 9b.

Vitamin C tested at 3% and 0.3% has significant levels of inhibition insofar as this molecule has a cytotoxic and non-specific action on the melanocytes.

Consequently, this molecule cannot be considered as active in our model.

The aurones prove to be very effective molecules on normal human tyrosinase.

Since the aurones are structurally close to flavones, a study consisting in testing flavones compared to aurones obtained preferably from Example 9b and 7a were made. Apigenin (structurally close to the derivative obtained in Example 9b) was tested at 10⁻⁴ M and 10⁻⁵ M. Chrysin (structurally close to the derivative obtained in Example 7) was tested at a concentration of 10⁻⁵ M. The results obtained are summarized in Table 11 below :

**Table 11 : Comparison with flavones**

| | | |
|---|---|---|
| | 10⁻⁴ M | 10⁻⁵ M |
| Example 9b | 75.31 ± 8.51 | 35.23 ± 2.34 |
| Apigenin | -59.06 ± 23.02 | -40.83 ± 7.68 |
| Example 7 | 3.37 ± 4.8 | 34.85 ± 3.04 |
| Chrysin | nd | -6.10 ± 22.87 |

It results from Table 11 that the aurone derivatives inhibit human tyrosinase specifically ; in fact, apigenin and chrysin tested at identical molar concentrations do not inhibit tyrosinase, the OD/protein ratio obtained being greater than the OD/protein ratio of the non-treated control.

The presence of a pentane ring in C instead of a hexane ring (in the case of flavones) seems to be indispensable in order to inhibit human tyrosinase. This enables the specificity of the aurones for the depigmenting activity sought after to be demonstrated.

### Example 20 of the invention: Example de formulation containing the aurones or aurone derivatives.

Use of the products of the invention in cosmetic or pharmaceutical formulations of oil-in-water type.

### Formulation 20a :

| | | |
|---|---|---|
| A | Water | qsp 100 |
| | Butylene Glycol | 2 |
| | Glycerine | 3 |
| | Sodium Dihydroxycetyl Phosphate, | 2 |
| | Isopropyl Hydroxycetyl Ether | |
| | | |
| B | Glycol Stearate SE | 14 |
| | Triisonoaoin | 5 |
| | Octyl Cocoate | 6 |
| | | |
| C | Butylene Glycol, | 2 |
| | Methylparaben, Ethylparaben, Propylparaben, pH adjusted to 5.5 | |
| | | |
| D | Products of the invention | 0.01 - 10 % |

### Formulation 20b :

| | | |
|---|---|---|
| A | Water | qsp 100 |
| | Butylene Glycol | 2 |
| | Glycerine | 3 |
| | Polyacrylamide, Isoparaffin, Laureth-7 | 2.8 |
| | | |
| B | Butylene Glycol, | 2 |
| | Methylparaben, Ethylparaben, Propylparaben ; | 2 |
| | Phenoxyethanol, Methylparaben, Propylparaben, Butylparaben, Ethylparaben | 0.5 |
| | Butylene Glycol | |
| | | |
| D | Products of the invention | 0.01 - 10 % |

### Formulation 20c :

| | | |
|---|---|---|
| A | Carbomer | 0.50 |
| | Propylene Glycol | 3 |
| | Glycerol | 5 |
| | water | qsp 100 |
| | | |
| B | Octyl Cocoate | 5 |
| | Bisabolol | 0.30 |
| | Dimethicone | 0.30 |
| | | |
| C | Sodium Hydroxide | 1.60 |
| | | |
| D | Phenoxyethanol, Methylparaben, Propylparaben, Butylparaben, Ethylparaben | 0.50 |
| E | Perfume | 0.30 |
| | | |
| F | Products of the invention | 0.01 - 10 % |

### Example 21 of the invention : Use of the products of the invention in a formulation of water-in-oil type :

| | | |
|---|---|---|
| A | PEG 30 dipolyhydroxystearate | - 3 |
| | Capric Triglycerides | 3 |
| | Cetearyl Octanoate | 4 |
| | Dibutyl Adipate | 3 |
| | Grape Seed Oil | 1.5 |
| | Jojoba Oil | 1.5 |
| | Phenoxyethanol, Methylparaben, Propylparaben, Butylparaben, Ethylparaben | 0.5 |
| | | |
| B | Glycerine | 3 |
| | Butylene Glycol | 3 |
| | Magnesium Sulfate | 0.5 |
| | EDTA | 0.05 |
| | water | qsp 100 |
| | | |
| C | Cyclomethicone | 1 |
| | Dimethicone | 1 |
| | | |
| D | Perfume | 0.3 |
| | | |
| E | Products of the invention | 0.01 - 10 % |

### Example 22 of the invention : Use of the products of the invention in a formulation of shampoo or shower gel type :

| | | |
|---|---|---|
| A | Xantham Gum | 0.8 |
| | water | qsp 100 |
| | | |
| B | Butylene Glycol, | 0.5 |
| | Methylparaben, Ethylparaben, Propylparaben Phenoxyethanol, | 0.5 |
| | Methylparaben, Propylparaben, Butylparaben, Ethylparaben | |
| | | |
| C | Citric acid | 0.8 |
| | | |
| D | Sodium Laureth Sulfate | 40.0 |
| | | |
| E | Product of the invention | 0.01 - 10 % |

### Example 23 of the invention : Use of the products of the invention in an aqueous gel formulation (eyeliners, slimmers, etc.)

| | | |
|---|---|---|
| A | water | qsp 100 |
| | Carbomer | 0.5 |
| | Butylene Glycol | 15 |
| | Phenoxyethanol, Methylparaben, Propylparaben, Butylparaben, Ethylparaben | 0.5 |
| | | |
| B | Products of the invention | 0.01 - 10 % |

### Example 24 of the invention : Toxicological studies of the products of the invention.

### Evaluation of the cosmetic acceptation of a preparation containing the subject of the invention

Toxicology tests were carried out on the compound obtained according to Example 9 incorporated at 10% in a 0.5% xanthan gum, by an ocular evaluation in the rabbit, by the study of the absence of abnormal toxicity by single oral administration in the rat and by the study of the sensitizing power in the guinea pig.

### Evaluation of the primary irritation of the skin in the rabbit :

The preparations described above were applied without dilution at the dose of 0.5 ml on the skin of 3 rabbits according to the method recommended by the OECD Directive in relation to the study of "the acute irritant/corrosive effect on the skin".

The products are classed according to the criteria defined in the Decision of 1/2/1982 published in the Official Journal of the French Republic (the *"JORF"*) of 21/02/82.

### Evaluation of the ocular irritation in the rabbit :

The preparations described above were instilled pure and in one batch at the rate of 0.1 ml in the eye of three rabbits according to the method recommended by the directive of the OECD NO. 405 of February 24, 1987, in relation to the study of "the acute irritant/corrosive effect on the eyes".

The results of this test enable concluding that the preparations can be considered as non-irritant for the eyes, in the sense of Directive 91/326 EEC used pure or without dilution.

### Test on the absence of abnormal toxicity by single oral administration in the rat :

The preparations described were administered in one batch orally at the dose of 5g/Kg of body weight, to 5 male rats and 5 female rats according to a protocol inspired from the Directive of the OECD No. 401 of February 24, 1987 and adapted to cosmetic products.

The LD0 and LD50 are found to be greater than 5,000 mg/Kg. The preparations tested are therefore not classed amongst the preparations which are dangerous by ingestion.

### Evaluation of the skin sensitisation potential in the guinea pig :

The preparations described are subjected to the maximization test described by Magnusson and Kligmann, a protocol which is in agreement with the directive line No. 406 of the OECD.

The preparations are classed as non-sensitizing by contact with the skin.

## Claims

1. A method of cosmetic depigmentation care, comprising topically applying, onto at least one body area to be depigmented selected from area of the skin, the hair or the integuments, an effectively depigmenting amount of at least one aurone component selected from:
a) an aurone of general chemical formula (I): 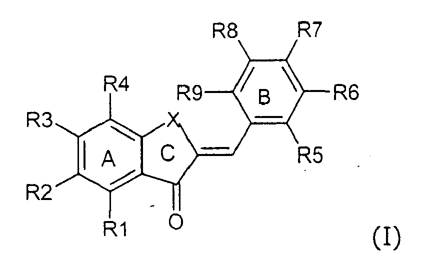 wherein X can represent an oxygen atom (O), a nitrogen atom (N) or a sulfur atom (S),
wherein R1, R2, R3, R4, R5, R6, R7, R8, R9 can represent, independently of each other : H ; a halogen ; a hydroxyl (OH) ; an ester ; a salified or non-salified C1-12 acid ; an amide ; a sulfonamide ; a nitro ; a basic or salified I, II or III amine ; a cyano ; a thiol ; a sugar (O-heteroside or C-heteroside) ; a linear or branched C1-12 alkyl chain ; a linear or branched C1-12 alkoxy chain ; a linear or branched C1-12 alkenyl chain (e.g. prenyl); C1-12 alkenyloxy ; a thioalkyl chain ; an alkyl or aromatic type ring or heterocycle ; a salified or non-salified sulfate ; a salified or non-salified sulfonate ; a salified or non-salified phosphate ; an esterified or non-esterified phosphate ; an esterified or non-esterified phosphonate; a salified or non-salified phosphonate, a silanol group of formula : 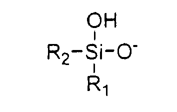 in which R1 and R2 can represent, independently of each other, a hydroxyl, methyl, ethyl, phenyl, *p*-methylphenyl, *p*-methoxyphenyl, or tert-butyl group;
b) an auronol, or azaaurone or thioaurone of the following chemical formula: 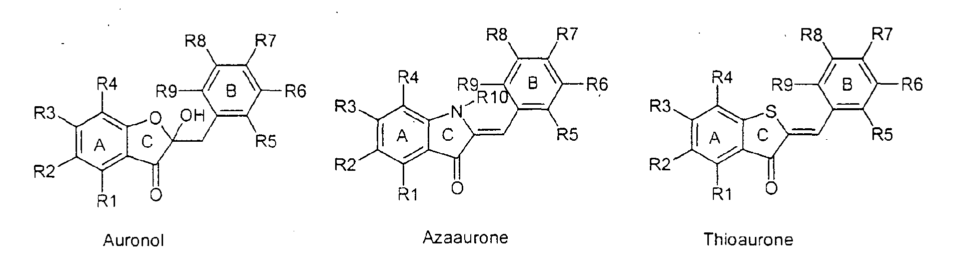 wherein R1-R9 represent, independently of each other, the same groups as defined for aurone under a) above, and R10 is selected from a linear or branched acyl group or alkyl group having 1 to 12 carbon atoms;
c) a dimer of aurone selected from disulfuretin and biaureusidin;
d) an aurone analogue obtained by replacing the independent phenyl ring, called the «B» ring of aurone as defoned under a) above by a heterocycle selected from the group consisting of pyrrole, imidazole, triazole, pyridine, furan, and thiophene of the following chemical formula: 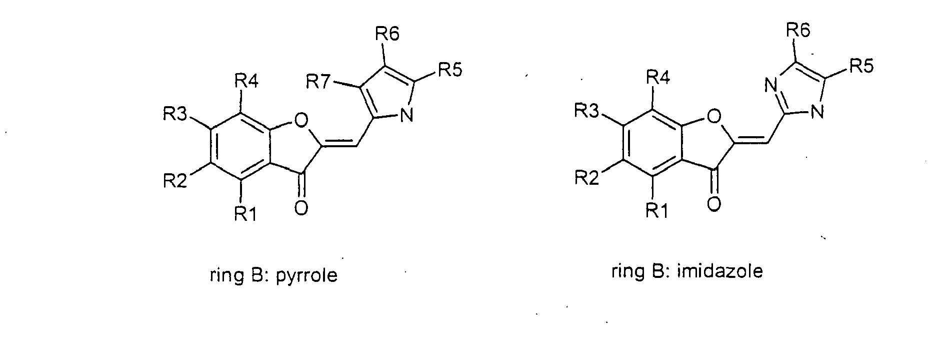 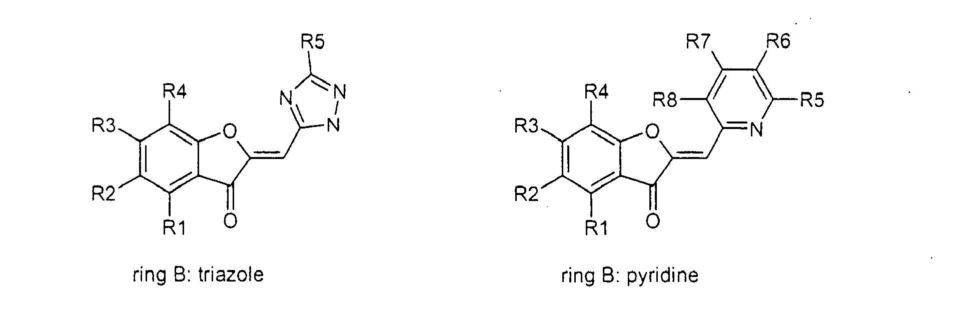 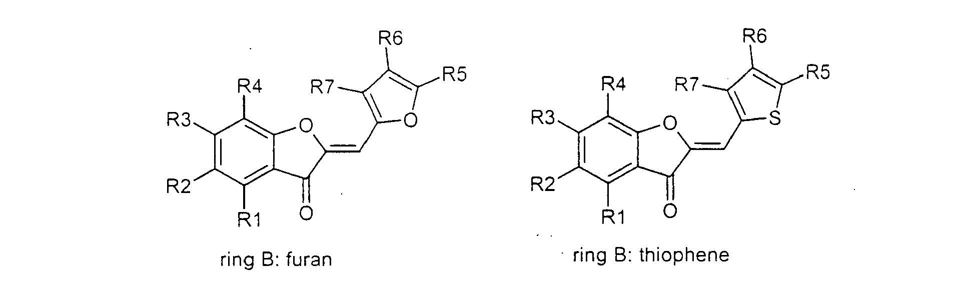
Wherein, R1 to R8 are as above defined;
optionally admixed with a cosmetically acceptable excipient, vehicle or support.

2. The method of claim 1, wherein the aurone component is selected from the group consisting of 2', 4', 6'-Trihydroxy-2-chloroacetophenone (R1 = OH,), 2',4'-Dihydroxy-2-chloroacetophenone (R1 = H) ; 4,6-Dihydroxybenzofuran-3(2H)-one (R1 = OH) ; 6-Hydroxybenzofuran-3(2H)-one (R1 = H) ; (Z)-2-Benzylidene-6-Hydroxybenzofuran-3(2H)-one ; (Z)-2-(4'-Ethyl)Benzylidene-6-Hydroxybenzofuran-3(2H)-one ; (Z)-2-(2'-Ethyl)-Benzylidene-6-Hydroxybenzofuran-3(2H)-one ; (Z)-2-(4'hydroxy)Benzylidene-6-Hydroxybenzofuran-3(2H)-one ; 4,6-Dimethoxybenzofuran-3(2H)-one ; (Z)-2-Benzylidene-4,6-dimethoxybenzofuran-3(2H)-one ; (Z)-2-(4'-hydroxy)Benzylidene-4,6-dimethoxybenzofuran-3(2H)-one ; (Z)-4,6-Dimethoxy-2-(4'-Ethylbenzylidene)benzofuran-3(2H)-one ; (Z)-4,6-Dimethoxy-2-(4'-methylthiobenzylidene)benzofuran-3(2H)-one ; (Z)-2-(4'-Cyanobenzylidene)-4,6-dimethoxybenzofuran-3(2H)-one ; (Z)-4,6-Dimethoxy-2-(4'-sulfonatebenzylidene)benzofuran-3(2H)-one ; (Z)-4,6-Dimethoxy-2-(2,4-disulfonatebenzylidene)benzofuran-3(2H)-one ; (Z)-4,6-Dimethoxy-2-(4'-benzylidene)benzofuran-3(2H)-one; (Z)-4,6-Dimethoxy-2-(4'-propylbenzylidene)benzofuran-3(2H)-one ; (Z)-4,6-Dimethoxy-2-(4'-isopropylbenzylidene)benzofuran-3(2H)-one ; (Z)-2-(4'-Butylbenzylidene)-4,6-dimethoxybenzofuran-3(2H)-one; (Z)-2-Benzylidene-(4,6-dihydroxy) benzofuran-3(2H)-one ; (Z)-4,6-Dihydroxy-2-(4'-ethoxybenzylidene)benzofuran-3(2H)-one ; (Z)-4,6-Dihydroxy-2-(4'-hydroxybenzylidene)benzofuran-3(2H)-one ; (Z)-4,6-Dihydroxy-2-(3-methoxy-4-hydroxybenzylidene)benzofuran-3(2H)-one ; 4,6-Di-OMEMbenzofuran-3(2H)-one ; 2,6-Diacetoxy-a-bromoacetophenone ; 4-Hydroxybenzofuran-3(2H)-one ; and (Z)-2-(4'-Hydroxybenzylidene)-4-Hydroxybenzofuran-3(2H)-one.

3. The method of claim 1, wherein the aurone component is selected from the group consisting of (Z)-4,6-Dihydroxy-2-(4-methoxybenzylidene)benzofuran-3(2H)-one ; (Z)-4,6-Dihydroxy-2-(4'-ethoxybenzylidene)benzofuran-3(2H)-one ; (Z)-4,6-Dihydroxy-2-(4'-hydroxybenzylidene)benzofuran-3(2H)-one; or (Z)-4,6-Dihydroxy-2-(3-methoxy-4-hydroxybenzylidene)benzofuran-3(2H)-one.

4. The method of claim 1, wherein the aurone component is selected from the group consisting of an aurone component in the form of an extract from a plant , and an aurone component obtained by chemical synthesis,

5. An aurone component selected from:
a) an aurone of the following chemical formula (I) : 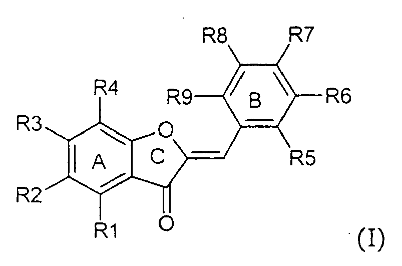 in which the R1 to R9 groups are as defined in claim 1, provided that at least one group from R5 to R9 is selected from a linear or branched C1-12 alkyl chain ; a linear or branched C1-12 alkoxy chain ; a C1-C12 thioalkyl group, a sulfonyl group, and a cyano group ;
b) an aurone analogue of the following chemical formula (II): 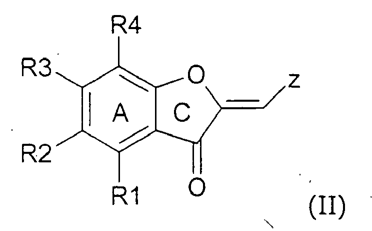 in which the R1, R2, R3, R4 groups are as defined above, and Z represents a heterocycle selected from the group consisting of pyrrole, pyridine, furan, and thiophene, which is non-substituted or substituted with a R5, R6, R7 group and optionally an R8 group as defined above.

6. The aurone component of claim 5, wherein at least one group from R5 to R8 is selected from a linear or branched C1-12 alkyl chain ; a linear or branched C1-12 alkoxy chain , a C1-C12 thioalkyl group, a sulfonyl group, and a cyano group.
